# EUROPEAN PATENT SPECIFICATION

(11) **EP 1 019 491 B1**
(45) Date of publication and mention of the grant of the patent: **07.12.2005**
(21) Application number: 98939102.4
(22) Date of filing: 04.08.1998
(51) Int. Cl.: C12N 5/00, C12N 5/02, C12N 15/00, C12N 5/06

(54) **PRODUCTION OF AVIAN EMBRYONIC GERM (EG) CELL LINES BY PROLONGED CULTURING OF PGCS, USE THEREOF FOR CLONING AND CHIMERIZATION**
HERSTELLUNG EMBRYONISCHER VOGEL KEIMZELLINIEN DURCH VERLÄNGERTE KULTIVIERUNG VON PGCS, VERWENDUNG DESSELBEN ZUR KLONIERUNG UND CHIMERISIERUNG
ELABORATION DE LIGNEES CELLULAIRES GERMINALES D'EMBRYON D'OISEAU PAR CULTURE PROLONGEE DE CELLULES SEXUELLES PRIMORDIALES, ET LEUR UTILISATION POUR LE CLONAGE ET LA PRODUCTION DE CHIMERES

(30) Priority: 04.08.1997 US 54677 P
(43) Date of publication of application: 19.07.2000
(73) Proprietor: University of Massachusetts, a Public Institution of Higher Education of The Commonwealth of Massachusetts,, Amherst, MA 01002 (US)
(72) Inventor: PONCE de LEON, F., Abel University of Minnesota, St. Paul, MN 55108 (US); ROBL, James, M., Belchertown, MA 01007 (US); STICE, Steven, L., Belchertown, MA 01007 (US); JERRY, D., Joseph, Shutesbury, MA 01072 (US)
(74) Representative: Campbell, Patrick John Henry
(86) International application number: PCT/US1998/015636
(87) International publication number: WO 1999/006534

(56) References cited:
- US-A- 5 453 357
- PONCE DE LEON F A ET AL: "Recent advances in chicken primordial germ cell technology" REVISTA BRASILEIRA DE REPRODUCAO ANIMAL,BR,BELO HORIZONTE, vol. 21, no. 3, 1997, pages 96-101, XP002913042 ISSN: 0102-0803
- PAIN B ET AL: "Long-term in vitro culture and characterisation of avian embryonic stem cells with multiple morphogenetic potentialities" DEVELOPMENT, vol. 122, August 1996 (1996-08), pages 2239-2248, XP002913043 ISSN: 0950-1991
- HAN J.Y. et al., "Primordial Germ Cells in Aves", AJAS, December 1994, Vol. 7, No. 4, pages 459-466, XP002913030
- PETITTE J.N. et al., "Culture of ESC-Like Cells from the Chicken Blastoderm", POULTRY SCIENCE, 1993, Vol. 72, Supplement 1, page 95, XP002913031

## Description

### FIELD OF THE INVENTION

The present invention provides a novel method for maintaining avian primordial germ cells (PGCs), in particular chicken PGCs, for prolonged periods in tissue culture which results in the production of embryonic germ (EG) cells. These EG cells can be used for the insertion of desired DNA sequences, e.g., human genes. These EG cells and transgenic EG cells derived therefrom, may be used to produce chimeric birds, in particular chimeric chickens, and for cloning.

### BACKGROUND OF THE INVENTION

In recent years there has been much research focused toward the production of chimeric, cloned and transgenic animals.

In particular, the modification of the genome of farm animal species is an area which has been actively pursued, with varying degrees of success, for the past two decades. For example, such research has been focused toward generating transgenic pigs, cows, and chickens. To date, the majority of the available transgenic animals have been generated by the direct microinjection of single cell embryos with DNA constructs harboring the gene of interest. However, while microinjection techniques have been successful, such methods are disadvantageous in that they are costly and often suffer from low efficiency.

Recently, the success of embryonic stem (ES) cell technology for the production of "knock-out" mice has led to research focused toward the development of tissue culture systems for ES cells and primordial germ cells (PGCs) in farm animal species. The ability to maintain undifferentiated ES cells in continuous culture enables *in vitro* transfection of such cells and ideally selection of transfected cells which contain a desired gene prior to their transfer to the inner cell mass of a developing embryo to generate chimeric animals. Ideally, at least some of the resultant chimeric animals will be able to segregate the DNA construct via the germ line and, hence, produce transgenic progeny. However, to date, targeted (site-specific) integrations have only been achieved in mice. Currently, the ability to do targeted DNA integration in other animal species is limited. However, work in this direction is in progress and should be realized soon.

In particular, there has been considerable research targeted toward improving the genome of Gallinacea and chickens in particular because of the considerable economic importance thereof. A fairly complete review of the state of research directed at the generation of transgenic chickens was published three years ago (Sang, *Trends in Biotech.,* 12:415-420 (1994)). As discussed therein, there are basically two alternative routes under investigation for producing transgenic chickens. These methods can be distinguished based on the time when manipulation of the genome is effected, i.e., before lay or after lay. The latter method includes the transfer of donor ES cells and PGCs to recipient embryos. Moreover, in both routes, the bulk of the work has been effected by infecting donor cells with retroviral vectors containing a gene of interest.

The first approach, which comprises manipulation of the genome before lay has yielded mixed and/or inefficient results. For example, the infection of oocytes in the ovary (Shuman, and Shoffner, Poultry Sci., 65:1437-1494 (1986) and pre-incubation of sperm with plasmid DNA (Gruenbaum et al., *J*. *Cell. Biochem Supp.,* 15:194 (1991) were inefficient and have not been repeated. Also, the transfection of sperm cells with a plasmid construct by lipofection has been demonstrated (Squires and Drake, *Anim. Biotech.,* 4:71-78 1993). However, germ line transmission was not reported.

Also, the direct microinjection of DNA into the germinal disk followed by embryo culture has been reported to yield 0.1% live transgenic chimeric birds (Sang, W., *Trends in Biotech.,* 12:415-42 (1994)) with one bird transmitting the transgene to 3.4% of its offspring (Love et al., *Bio*/*Technology*, 12:60-63 (1994)). This same approach was taken by Naito et al *(J. Reprod. Fertil.,* 102:321-325 (1994)). However, similarly no germ line transmission of the transgene was reported therein.

The second approach, which comprises manipulation of the genome after lay, has yielded better results. Chimeric birds, generated by injection of laid eggs with replication competent retroviral vectors, have shown germ line transmission to 1% and 11% of their offspring (Salter et al., *In Manipulation of the Avian Genome,* Etches, RJ et al., eds. pp 138-150 CRC Press (1993)). More encouraging results, using replication-defective retroviral vectors and injection into laid eggs, generated 8% chimeric male birds that transmitted the vector to their offspring at a frequency of 2 to 8% (Bosselman et al., *Science,* 243:535-535 (1989)).

However, the injection of laid eggs with plasmid constructs in the presence of reagents known to promote transfection has failed to yield stably integrated constructs or transgenic birds (Rosenblum and Cheng, J., *Cell Biochem Supp.,* 15E 208 (1991)). In general, the use of retroviral vectors for the generation of transgenic chickens is not widespread because of significant disadvantages associated therewith. Such disadvantages include the constraints on the size of the cloning insert that can be stably introduced therein and the more serious potential disadvantage of possibly inducing recombination events with endogenous viral loci or with other avian leukosis viruses.

A significant problem with all of these methods is the fact that long term culture systems for chicken ES cells and PGCs have been relatively difficult to establish. To the best of the inventors' knowledge, it is believed that the longest avian PGCs have been cultured with the successful production of chimeric birds is less than 5 days.

Previous PGC culturing methods have included the use of growth factor, in particular LIF or IGF. However, as noted, such methods have not been able to provide for prolonged culturing periods, a prevalent concern as it would facilitate the production of transgenic PGCs.

However, notwithstanding the problems in achieving long term culturing, both ES cells and PGCs have been successfully used to generate chimeras by infection of such cells with replication competent and incompetent retroviral vectors. Further, as discussed above, freshly obtained blastodermal cells have been injected into recipient embryos, resulting in birds with chimeric gonads (Carsience et al., Devel., 117:669-675 1993)). Blastodermal cells can be efficiently transfected by lipofection and then transferred into recipient embryos. However, germ line transmission of transfected cells has not been reported.

Also, Pain et al., Devel., 122:2329-2398 (1996), have recently demonstrated the presence of putative chicken ES cells obtained from blastodermal cells. They further reported maintenance of these cells in cultures for 35 passages assertedly without loss of the ES phenotype (as defined by monoclonal antibodies to mouse ES cells). (*Id*.) These cells apparently develop into PGCs upon transfer into avian embryos where they colonize in the gonads. However, they did not establish definitively that these cells were in fact ES cells.

The cross-reactivity of mouse ES monoclonal antibodies with chicken ES cells might argue favorably for conservation of ES cell receptors across species. Also, the fact that these researchers were also able to generate two chimeric chickens with injections of 7 day old blastodermal cell cultures would arguably suggest the presence of ES cells in their system. However, these researchers did not rule out the possibility that PGCs were present in their complex culture system. Thus, this long term ES culture system should be further tested for piuripotency and germ line transmission. (Id.)

An alternative route to the production ES cells, comprises PGCs. Procedures for the isolation and transfer of PGCs from donor to recipient embryos have been developed and have successfully generated chimeric chicken with germ line transmission of the donor genotype (Vick et al., *London Ser. B.,* 251:179-182 (1993), Tajima et al., *Theriogenology*, 40:509-519 (1993)). Further, PGCs have been cryopreserved and later thawed to generate chimeric birds (Naito et al., *J. Reprod. Fertil.,* 102:321-325 (1994)). However, this system is very labor intensive and only yields, on average, only 50 to 80 PGCs per embryo. Infection of PGCs with retroviral vectors has also been reported. However, to date, the growth of PGCs in culture for prolonged periods to facilitate selection of transfected PGCs has not been achieved.

Thus, based on the foregoing, it is clear that improved methods for culturing PGCs comprises a significant need in the art. Also, another significant need comprises novel methods for producing avian embryonic stem (ES) or embryonic germ (EG) cells because of their application in the production of cloned avians and for the production of chimeric avians, and transgenic forms thereof.

### OBJECTS OF THE INVENTION

It is an object of the invention to solve the problems of the prior art.

It is a more specific object of the invention to provide a novel method for culturing avian primordial germ cells (PGCs) for prolonged periods in tissue culture which results in the production of embryonic germ (EG) cell lines.

Accordingly, the invention provides a culturing method which provides for the production of avian embryonic germ (EG) cells comprising the following steps:
(i) isolating primordial germ cells (PGCs) from a desired avian;
(ii) culturing said primordial germ cells in a culture medium containing at least the following growth factors contained in amounts sufficient to maintain said PGCs for prolonged periods in tissue culture:
   (1) leukemia inhibitory factor (LIF),
   (2) basic fibroblast growth factor (bFGF),
   (3) stem cell factor (SCF), and
   (4) insulin-like growth factor (IGF), for prolonged time period sufficient to produce a culture having a compact multilayer like appearance; and
(iii)identifying embryonic germ cells contained therein. The invention also provides an avian EG cell line obtained by the method of the invention.

It is an even more specific object of the invention to provide a novel method for culturing *Gallinacea,* especially chicken or turkey, primordial germ cells (PGCs) for prolonged periods in tissue culture to produce embryonic germ (EG) cell lines.

It is another object of the invention to use avian embryonic germ cells which have been obtained by culture of PGCs for prolonged periods in tissue culture for the production of chimeric avians, preferably poultry, and most preferably chickens.

Accordingly, the invention also provides a method of producing chimeric avians comprising:
(i) isolating primordial germ cells (PGCs) from an avian;
(ii) maintaining such PGCs in a tissue culture medium containing at least the following growth factors:
   (1) leukemia inhibitory factor (LIF),
   (2) basic fibroblast growth factor (bFGF),
   (3) stem cell factor (SCF), and
   (4) insulin-like growth factor (IGF)for a sufficient time to produce embryonic germ (EG) cells;
(iii)transferring said EG cells into a recipient avian embryo; and
(iv) selecting for chimeric avians which have the desired PGC phenotype.

It is another object of the invention to introduce desired nucleic acid sequences into avian embryonic germ cells which have been obtained by culture of avian primordial germ cells for prolonged periods in tissue culture.

It is yet another object of the invention to use avian germ cells, which have been produced by culturing primordial germ cells in culture for prolonged periods, into which a desired nucleic acid sequence has been introduced, for the production of transgenic chimeric avians, preferably transgenic chimeric chickens.

It is still another object of the invention to use the resultant transgenic chimeric avians, preferably *Gallinacea* and most preferably chickens or turkeys, for the production of heterologous protein(s) encoded by a nucleic acid sequence contained in cells introduced therein, preferably by recovery of such protein(s) from the eggs of such transgenic chimeric avians, in particular transgenic chimeric chickens and their progeny. Alternatively, such protein(s) can be recovered from the transgenic chimeric avian directly, e.g., from the circulatory system (blood or lymph) or other tissues, or body fluids.

It is another object of the invention to use avian germ cells, preferably chicken embryonic germ cells obtained by prolonged culturing of avian primordial germ cells for cloning of avians, e.g., cloned chickens (which may be transgenic).

### BRIEF DESCRIPTION OF THE DRAWINGS

**Figure 1**. EMA-1 antibody staining on mouse ES cells. Panels A and B denote two different cultures. A1 and B1 - DAPI stained images of mouse ES cell clusters. A2 and B2 - Phase contrast images of mouse ES cell clusters. A3 and B3 - Positive FITC signal on mouse ES cells.
**Figure 2.** EMA-1 antibody staining on 98-day old PGC cultures. Panels A and B denote two different clusters. Al and B1 - DAPI stained images of 98-day old PGC clusters. A2 and B2 - Phase contrast images the PGC clusters. A3 and B3 - Positive FITC signal on PGCs.
**Figure 3.** EMA-1 antibody staining on freshly collected chicken PGCS. Panels A and B denote two different treatments. A1 and B1 - DAPI stained images of fresh PGCs. A2 and B2 - Positive FITC signal on PGCs. Note arrow-heads on DAPI stained PGCs in A1 that correspond to PGCs showing positive FITC signal in A2.
**Figure 4.** EMA-1 antibody staining on chicken primary fibroblast cells. Panels A and B denote two different cultures. A1 and B1 - DAPI stained images of chicken fibroblasts. A2 and B2 - Phase contrast images of chicken fibroblasts. A3 and B3 - FITC image of chicken fibroblasts (negative).
**Figure 5.** MC-480 antibody staining on mouse ES cells. Panels A and B denote two different cultures. A1 and B1 - DAPI stained images of ES cell cluster. A2 and B2 - Phase contrast images of mouse ES cells. A3 and B3 - Positive FITC signal on mouse ES cells.
**Figure 6.** MC-480 antibody staining on one treatment of a 98-day old PGC culture. A1 - DAPI stained image of 98-day old PGC cluster. A2 - Phase contrast image of the PGC cluster. A3 - Positive FITC signal on 98-day old PGCs.
**Figure 7.** MC-480 antibody staining on freshly collected chicken PGCs. Panels A and B denote two different treatments. A1 and B1 - DAPI stained fresh PGCs. A2 and B2 - Positive FITC signal on PGCs. See arrow-heads on DAPI-stained PGCs in A1 corresponding to positive FITC signals in A2.
**Figure 8.** MC-480 antibody staining on chicken primary fibroblast cells. Panels A and B denote two different cultures. A1 and B1 - DAPI stained images of chicken fibroblasts. A2 and B2 - Phase contrast images of chicken fibroblasts. A3 and B3 - FITC image on chicken fibroblasts (negative).

### BRIEF DESCRIPTION OF THE INVENTION

As discussed, the present invention provides a novel method for maintaining avian (e.g. chicken) primordial germ cells (PGCs) in tissue culture for prolonged periods, i.e., for at least 14 days, more preferably at least 25 days, and ideally indefinitely. We are now at 4 months of continuous culture and approximately 32 cell passages.

Prior to the present invention, there were not reported any methods for maintaining avian PGCs in tissue culture which provided for their maintenance for longer than about 5 days (as demonstrated by their ability to produce chimeric avians). The present inventors have surprisingly discovered, by judicious experimentation, that the use of a culture media containing at the least the following growth factors: leukemia inhibitory factor (LIF), basic fibroblast growth factor (bFGF), stem cell factor (SCF) and insulin-like growth factor (IGF) enables avian primordial germ cells, specifically chicken primordial germ cells to be maintained and to proliferate for prolonged periods, i.e., at least 14 days, and for substantially longer in tissue culture. Moreover, these PGCs have been demonstrated to be useful for the generation of chimeric chickens.

These PGCs are useful for the production of transgenic avian PGCs, which can be used to produce transgenic chimeric avians. It is expected that these transgenic chimeric avians will be useful for recovery of heterologous proteins, which preferably can be recovered directly from the eggs of such chimeric transgenic avians, or from tissues and other body fluids. For example, such avians can be used for the production and recovery of therapeutic proteins and other polypeptides.

However, the basis of this invention is the further observation that these PGCs, after prolonged culturing, i.e., about after 25 days, de-differentiate, and apparently result in the production of embryonic germ (EG) cells.

Specifically, after 25 days, the cultured PGCs (clumps) form rapidly spreading cell monolayers which have a flat adherent base. On the surface thereof are looser "PGC-Like" cells. Moreover, some of these cells stain PAS positive. Also, DiI stained cells obtained from these monolayers, upon transfer to recipient avian embryos, localize in the gonads. Moreover, these cell monolayers can be passaged theoretically indefinitely.

It was also observed that after about 3 to 5 passages, some cells slow down in their role of proliferation and appear fibroblast-like in appearance. However, some cell lines have been passaged multiple times, and continue to thrive without any signs of differentiation, even after four months in continuous tissue culture. It was also observed that, as the number of cells increases in such cell colonies, the cell monolayer becomes more "compact", giving the appearance of multilayer cell colonies.

As discussed *infra,* two cell lines have been obtained therefrom, one of which is positive for alkaline phosphatase and apparently is not differentiated. Also, it expresses other markers characteristic of pluripotent and totipotent cell types. Thus, it is believed that this cell line is an embryonic germ cell line. Thus, this invention is based on the discovery that PGCs can de-differentiate in culture to produce EG cells.

As discussed infra, this is a very significant discovery as such cells can be used for cloning avians, and for producing chimeric avians. Also, these embryonic germ cells can be used to study the differentiation of avian embryonic cell lines in vitro. Still further, these cells can be rendered transgenic (by introduction of desired nucleic acid sequence) and used to make transgenic chimeric or cloned avians, preferably of the genus *Gallinacea*, and most preferably chickens or turkeys.

### DETAILED DESCRIPTION OF THE INVENTION

Thus, the present invention obviates the problems associated with previous avian PGC culturing methods which did not enable such PGCs to be maintained in tissue culture for periods longer than about five days. As discussed in detail infra, the present inventors have surprisingly discovered that avian PGCs, preferably *Gallinacea* PGCs, and most preferably chicken PGCs, can be maintained in tissue culture for prolonged periods, in at least 14 days, more preferably at least 25 days, and preferably longer, by the use of culture medium which contain at least the following four growth factors:
leukemia inhibiting factor (LIF), stem cell factor (SCF), insulin-like growth factor (IGF) and basic fibroblast growth factor (bFGF).

In general, such culturing method comprises the following steps:
(i) isolating PGCs from donor Stage XII to XIV avian embryos; and
(ii) culturing said isolated avian PGCs in a culture medium containing relative amounts of LIF, bFGF, SCF and IGF effective to promote their proliferation, for a prolonged time, i.e., typically after at least 28 days, in tissue culture to produce EG cells. Moreover, as discussed supra, the present invention is based on the discovery that such PGCs, after being cultured in this medium for prolonged periods, on average at about 25 days, apparently de-differentiate to produce avian embryonic germ cells. In this regard, it has been earlier reported that mouse PGCs maintained on STO feeder cell monolayers in the presence of LIF and bFGF resulted in cells resembling embryonic stem cells (Resnick et al, *Nature*, 359:550-551, 1992; Matsui et al, Cell, 70:841-843, 1992). Resnick et al (*Id*.) suggested the name of embryonic germ (EG) cells for this type of cell, to imply that they originated from PGCs in vitro, although it was not clear at the time whether EG cells were significantly different than traditional ES cells.

It has since been shown, at least with mouse embryonic cell lines, that EG cells differ from ES cells in the methylation state of certain genes (Labosky et al., Development, 120: 3197-3204, 1994; Piedra hita et al., Biology of Reproduction, 58: 1321-1329, 1998). However, like ES cells, EG cells have been shown to differentiate extensively in culture and contribute to chimeras when injected into host blastocysts, thus demonstrating their pluripotent and totipotent nature. It remains to be shown whether avian EG and ES cells will also have differences in gene methylation. Although not wishing to be held to this hypothesis, the cells of the present invention are believed to be EG cells because they are derived from PGC's and not from the blastoderm as are ES cells.

The fact that these cells are apparently embryonic germ cells is supported by various tests. In particular, tissue cells are positive for alkaline phosphatase (Pain et al, Development, 122:2339-2342, 1996), and mouse-specific antigens 1 and 3 (based on reactivity with monoclonal antibodies specific for SSEA-1 and SSEA-3). These are markers for pluripotent and totipotent cells. Thus, avian (e.g. chicken) and mouse pluripotent and totipotent stem cells apparently share related epitopes, characteristic of their undifferentiated state. Thus, these antibodies are useful for selecting avian embryonic germ cells which arise in cell colonies produced upon prolonged culturing of avian PGCs using the subject culture system.

The totipotency and pluripotency of these EG cells can be confirmed by transferral to stage X chicken embryos (as described by Etches et al, Poultry *Science,* ₇2:882-887, 1993). This will provide evidence that these avian EG cells are capable of giving rise to different tissues characteristics of different developmental stages (pluripotent) as well as migrating to the gonads, demonstrating germ-line transmission. Therefore, after transfer, these EG cells should give rise to somatic and germ line chimeric birds.

Methods for isolation of primordial germ cells from donor avian embryos have been reported in the literature and can be effected by one skilled in the art. (See, e.g., JP 924997 published September 7, 1993 Pub. No. 05-227947; Chang et al., *Cell Biol. Int.,* 19(2):143-149 (1992); Naito et al., *Mol. Reprod. Devel.,* 39:153-161 (1994); Yasuda et al., *J. Reprod. Fert*., 96:521-528 (1992); and Chang et al., *Cell Biol. Int. Reporter,* 16(9):853-857 (1992), all of which are incorporated by reference in their entirety therein).

The present inventors elected to isolate avian PGCs from chicken eggs which had been incubated for about 53 hours (stage 12-14 of embryonic development), removal of embryos therefrom, collection of embryonic blood from the dorsal aorta thereof, and transferral thereof to suitable cell culture medium (M199 medium). These PGCs were then purified by ficoll density centrifugation, and resuspended in 10µl of the growth factor containing culture medium of the present invention. However, as discussed above, other methods for isolating PGCs are known and may alternatively be used.

The isolated PGCs are then counted and separated manually (e.g., using a pipette). Thereafter, PGCs collected from these different avian embryos are pooled (to increase PGC numbers) and incubated in the subject growth factor containing medium.

This culture medium, hereinafter referred to as "complete" medium contains LIF, bFGF, SCF and IGF as well as other substituents typically comprised in PGC and embryonic stem cell medium. More specifically, the subject "complete" medium will preferably comprise α-MEM, a well known commercially available cell growth medium to which has been added the above four growth factors and which additionally includes 10% fetal calf serum, 2 mM L-glutamine, 0.48% antibiotic/antimitotic, 132 µM 2-β mercaptoethanol,1 U/µl of LIF, 0.40 pg/µl of bFGF, 60 pg/µl of IGF-I and 80 pg/µl of SCF.

Based on the experiments conducted to date, these are believed to correspond to the minimal concentrations of these growth factors. However, as described infra, the amounts of these growth factors have been doubled with.PGCs being successfully maintained in tissue culture. Thus, it is known that the respective amounts of these growth factors may be increased with no adverse effects. Moreover, even these minimum amounts may vary, e.g., if PGCs of other avians are cultured.

As noted, the present inventors used as the base medium, α-MEM, a well known commercially available tissue culture medium. However, it is expected that other media may be substituted therefor, provided that these four essential growth factors are also present. Applicants particularly contemplate modification of the subject "complete media" to eliminate fetal calf serum, because of its undefined and variable composition.

A particular advantage of the present invention is the fact that the EG cells may be maintained in the absence of a feeder layer, which provides for purer colonies and a cleaner preparation when producing chimeric or cloned animals. The increased purity of the EG cell preparation in turn results in an increased probability of success in producing chimeric and cloned animals. However, the present invention may also be performed with a feeder layer provided these cells are transfected with genes encoding the disclosed growth factors, thereby eliminating the need for the exogenous addition of these factors during culturing. Essentially, the cells will provide a continual source of these growth factors. (This will be achieved by placing these growth factor genes under control of constitutive strong promoter and also sequences that provide for the secretion thereof, thereby making these growth factors available to cultured PGCs.)

As noted, the amounts of these factors refer to relative amounts thereof effective to enable prolonged culturing of avian PGCs, preferably Gallinacea PGCs, and most preferably chicken or turkey PGCs, for prolonged periods in tissue culture. In the present invention, this further refers to amounts that give rise to de-differentiation of the cultured PGCs into EG cells.

Preferably, the relative amounts of these growth factors will fall within the following ranges:
LIF 1 U/µl to 100 U/µl, more preferably 1 to 10 U/µl and most preferably 1 to 5 U/µl;
IGF-I 0.60 pg/µl to 60.00 pg/µl, more preferably 0.60 pg/µl to 6.0 pg/µl by weight and most preferably 0.60 pg/µl to 1.0 pg/µl;
SCF 80 pg/µl to 8000 pg/µl by weight, more preferably 80 pg/µl to 800 pg/µl and most preferably 80 pg/µl to 160 pg/µl by weight; and
bFGF 0.40 pg/µl to 40 pg/µl, more preferably 0.40 pg/µl to 4.0 pg/µl by weight and most preferably 0.40 pg/µl to 0.80 pg/µl.

In the ranges set forth above, the upper ranges are not critical to the invention and are largely dictated by cost (given the significant expense associated with manufacture of growth factors).

However, it is expected that these preferred ranges may vary, e.g., if α-MEM is substituted by another growth medium and if other types of avian PGCs are cultured.

As discussed, these PGCs can be maintained for long periods in culture with successful production of chimeric avians. To date, the cells have been maintained in tissue culture for up to about 4 months, with apparently no adverse effects. Also, cells of up to 25 days have been tested for their ability to effectively colonize avian embryonic gonads and produce chimeric birds. However, it is expected that these cells can be cultured indefinitely, with retention of the ability to produce chimeric birds.

Methods for using PGCs to produce chimeras are known in the art as evidenced by the prior art discussed *supra.* Preferably, EG cells will be transferred into recipient avian embryos according to the methods disclosed in the example that follows. Thereafter, successful chimera production is evaluated based on migration and colonization of PGCs in the gonads, retention of PGC phenotype, or by evaluating for the presence of donor PGCs in gonads after hatching and breeding.

In the present example, the inventors selected genotypes which are easily followed which affect coloration. (Donor birds were white broiler type and recipient birds were black feathered birds, respectively, having specific potential genotypes.) Putative chimeras were black feathered and produced black/white progeny when they were mated with black feathered birds. Thereby, successful chimeras were demonstrated based on the production of black/white feather containing birds.

In a second strategy Bar Rock birds were used as recipients, and white feathered birds used as donors. Putative chimeric birds were demonstrated based on the production of white feathered progeny having some barred feathers.

However, the subject method should be applicable for introducing any desired trait by chimerization. This will, of course, depend on the genotypic properties of the transferred PGCs.

As discussed, a significant application of the subject PGCs, which can be maintained in culture for long periods, is for the production of chimeric avians. This will be accomplished by introducing a desired DNA sequence into the cultured PGCs. Means for introducing DNAs into recipient cells are known and include lipofection, transfection, microinjection, transformation, microprojectile techniques, etc. In particular, the present inventors initially elected to introduce a vector containing a reporter gene by lipofection. However, while transiently transfected PGCs were produced, a stable transfected cell line has not, as yet, been isolated. However, it is expected that this can be accomplished by known techniques using the subject PGCs.

Preferably, a DNA will be introduced that encodes a desired gene, e.g., therapeutic polypeptide, growth factor, enzyme, etc., under the regulatory control of sequences operable in avians. Preferably, these regulatory sequences will be of eukaryotic origin, most preferably avian, e.g., chicken regulatory sequences. Promoters operable in avian cells, e.g., derived from avian genes or viruses are known in the art.

Initially, a stable cell line which produces the desired protein will be isolated and used for chimera production. Also, it is desirable that the introduced DNA contain a marker DNA, the expression of which is easily detected, to more easily identify cells containing the inserted DNA. Such selectable markers are well known and include β-lactamase, β-galactosidase, neomycin phosphotranspherase, etc.

Injection of the resultant transgenic PGCs into avian embryos will then result in the production of transgenic chimeric avians. Preferably, the desired protein will then be recovered from the eggs body fluids etc. of these transgenic avians, thereby providing a continual supply of the protein.

As discussed, the present invention involves the production of EG cells from PGCs which have been cultured as described above.

These EG cells will be identified based on their expression of characteristic "ES" antigens or markers, in particular alkaline phosphatase and stage-specific embryonic antigens. For example, monoclonal antibodies specific for SSEA-1 and SSEA-3 can be used to identify pluripotent and totipotent cells in PGCs which have been cultured for prolonged periods, typically at least 25 days in tissue culture. MC-480, for example, is a monoclonal antibody specific for the SSEA-1 antigen (Solter and Knowles (1978)).

Also, another monoclonal antibody, EMA-1, is specific for mouse and chicken PGCs and thereby should allow the identification of PGC cultures that retain PGC-specific epitopes. (This antibody binds specific epitopes expressed on both mouse and chicken promodial germ cells.) Therefore, EMA-1 should be useful for further characterization of avian EG cells generically, since these epitopes are apparently conserved in very different species (avians and mammals).

As discussed, the totipotency and pluripotency of these EG cells can be tested by transferral to avian embryos, e.g., by transferral to stage X chicken embryos as disclosed by Etches et al, Poultry *Science,* 72:882-889, 1993 and stage XII-XIV embryos as discussed above. This will provide experimental evidence that these EG cells differentiate into different tissue types (pluripotent) found in developing embryo and also that they successfully migrate and colonize the gonads (demonstrates that such cells will be transmitted to the germ line). Therefore, these cells will result in somatic and germ line chimeric birds, e.g., chimeric chickens.

### GENERATION OF TRANSGENIC CHICKEN:

Development of a culture system to support the proliferation of PGCs and further allow their de-differentiation into EG cells increases our ability to transfect cells with DNA vector constructs carrying exogenous genes for the systemic production of foreign proteins in chickens. Similarly, the generation of site directed (homologous recombination) also known as "knock-outs" and "knock-ins" transgenic chickens will be possible since the method facilitates selection and proliferation of EG cells after transfection.

### USE OF EG CELLS FOR CHICKEN CLONING:

Cloning of mammals has already been achieved. Cloning of birds can be effected using PGCs and EG cells and possibly differentiated embryonic cells (chicken embryonic fibroblasts, CEF). This can be accomplished as follows:
1. Chicken chimeras will be produced by gamma irradiation of freshly laid eggs in such a way that the cells of the embryo are compromised. This will be followed by microinjection of cloned EG cells in numbers approximately equivalent to the number of cells contained in the compromised blastoderm. The optimum level of gamma irradiation and the number of injected cells may be readily determined according to teachings in the art (Carsience et al., Development (1993) 117:659-675; Etches et al., Poultry Sci. (1993)73:882-889.)
2. Chicken clones will be generated from freshly laid unfertilized eggs, by extraction of the unfertilized oocyte followed by gamma irradiation, electrical stimulation of the oocyte, injection and fusion of an EG, PGC or CEF. After fusion, the oocyte will be transferred to a petri dish containing embryo culture media (Ono et al, *Devel. Biol.,* 161:126-130, 1994), or grafted back into an unfertilized egg.

### EXAMPLE

The following materials and methods were used in the experiments described below.

### Materials and methods.

### Monoclonal Antibodies

Primary antibodies EMA-1 and MC-480 (anti-SSEA-1 antibody) were obtained from Developmental Studies Hybridoma Bank (DSHB), The University of Iowa.

### EMA-1 antibody:

Monoclonal antibody EMA-1 is a cell surface marker specific for mouse primordial germ cells (PGCs), developed by Hahnel and Eddy (1986). This reagent was developed against the cell surface markers of Nulli SCCI mouse embryonal carcinoma (EC) cells. The antibody was prepared by fusing NS-1 myeloma cells with spleen cells from C57BI/6J mice immunized with Nulli SCCI EC cells. EMA-1 monoclonal antibody is of IgM isotype (Addendum # 1). The antigen recognized by the antibody is a cell surface glycoprotein. The expression of EMA-1 antigen on mouse PGCs is restricted to days 8 through 13 in a developing mouse embryo. EMA-1 reacts with most but not all pluripotent cells in early embryos (Hahnel and Eddy, 1987). According to Hahnel and Eddy (1986), PGCs are the only cells that stained strongly with EMA-1 in the caudal regions of 9.5 to 11-day embryos. It showed reactivity with PGCs in the urogenital ridges of the caudal half region of 13-day old embryo sections of male mouse. It did not show reactivity with PGCs in 14-day old mouse embryo sections. EMA-1 binds to the periphery and to a cytoplasmic granule present in PGCs. The antigen carrying EMA-1 determinant on the Nulli cells is insensitive to EDTA and trypsin treatment.

### MC-480 (Anti-SSEA-1) antibody:

Monoclonal antibody MC-480 recognizes a stage specific mouse embryonic antigen SSEA-1. The antibody is of the isotype IgM, described by Solter and Knowles (1978). The cell surface antigen SSEA-1 identified by this antibody is composed of a carbohydrate epitope on glycolipids and glycoproteins involving fucosylated type 2 blood group (addendum #2). The antibody was developed by the fusion of mouse myeloma cells with spleen cells from mouse immunized with F9 teratocarcinoma cells. The specificity of this antibody was tested on a series of mouse and human cell lines using radioimmunoassay (RIA). The antibody reacted with mouse teratocarcinoma cells and two human teratocarcinoma-derived cell lines (Solter and Knowles, 1978). All differentiated cell lines derived from the same tumors and teratocarcinoma stem cell lines were negative for the antigen. The supernatant from the hybridoma was further tested on mouse embryos. The antibody did not show reactivity with unfertilized eggs, zygotes, and 2- to 4-cell stage embryos. The antibody binds with increasing efficiency to late 8-cell stage embryos and morulae. The amount of binding decreased on blastocysts. Tests using complement dependent lysis showed a similar trend. No lysis of embryos prior to 8-cell stage was observed. Moderate lysis of 8-cell stage embryos (10-20%) was observed while morulae, blastocysts and inner cell masses lysed with high efficiency (Solter and Knowles, 1978). Results with indirect immunofluorescence assays also were similar where unfertilized eggs, zygotes and 2- and 4-stage embryos were negative. The majority of inner cell masses (ICM) cultured *in vitro* up to 3 days were positive for the antigen. Ectoderm exposed by removing the outer layer of endoderm from ICM grown *in vitro* was always completely positive. Solter and Knowles (1978) argued that probably several stage-specific glycosyl transferases are synthesized or activated and presented on cell surfaces during early preimplantation and embryonic development.

### Animals

White (E/E and I/I) broiler type chickens have been used as donors of PGCs to develop the long term PGC culture system. Two types of bird were used as recipient embryos, a dominant black feather (E/- and i/i) chicken line and a Bar Rock (E/E and i/i) line. Dominant black birds injected with white broiler (WB) type PGCs are referred as E/-(WB) and Bar Rock birds injected with white broiler type PGCs are referred as BR(WB).

### Extraction of PGCs

Stage 12 to 14 embryos were selected for PGC extraction. PGCs were collected from the dorsal aorta with a fine micropipette as described by Naito et al., *Mol. Reprod. Dev*., 37:167-171 (1994). PGCs from 20 embryos were pooled in Hanks' solution supplemented with 10% fetal bovine serum and concentrated by Ficoll density gradient centrifugation (Naito et al., *Mol. Reprod. Dev.,* 39:153-171 1994). PGCs were counted and distributed in 10 µl drops of culture medium (DMEM, containing differing amounts of growth factors) at about 100 to 600 PGCs per drop. Culture drops were overlaid with sterile light mineral oil.

### Injection of PGCs into recipient embryos.

Stage 13-14 embryos were used as recipient embryos. After placing the egg on an appropriate surface, time was allowed for the developing embryo to position itself on the upper side of the resting egg. A small 10 mm or less opening ("window") in the shell was made with a fine forceps. The embryo was brought close to the surface by adding a mixture of phosphate buffered saline (PBS) with 4% antibiotics. After accommodating the embryo to visualize its heart, the dorsal aorta and/or marginal vein could be easily identified. Two hundred donor PGCs in 2 µl of media containing 0.04% trypan blue were taken into a micropipette. PGCs were injected into the dorsal aorta of the recipient embryo. Trypan blue, an inert cell dye, allowed visualization of the PGC suspension when it was being delivered. After injection the egg shell opening was closed with surgical tape and reinforced with paraffin. Eggs were maintained for 24 hours under surveillance in a humidified CO₂ incubator and later transferred to a regular incubator until hatching.

### Viable fluorescent staining of PGCs.

To evaluate the success of transfers and/or the ability of PGCs to migrate to the gonads, PGCs were stained with DiI fluorescent stain. Embryos were collected after 24 hours of transfer, placed on a petri-dish and observed under an inverted microscope equipped for epi-fluorescent analysis.

### PGC culture conditions.

Several concentrations of human leukemia inhibitory factor (Lif), human basic fibroblast growth factor (b-FGF), human insulin-like growth factor (IGF) and human stem cell factor (SCF) have been tested. Likewise, mitomycin treated chicken fibroblast and mouse STO cell feeder layers were tested.

### PGC LONG-TERM CELL CULTURE MEDIUM.

The complete cell culture medium is made of α-MEM, 10% fetal calf serum, 2 mM L-glutamine, 0.56% antibiotic/antimitotic, 34.56 mM 2-β mercaptoethanol, 0.00625 U/µl of leukemia inhibitory factor (LIF), 0.25 pg/µl of basic fibroblast growth factor (b-FGF), 0.5625 pg/µl of insulin like growth factor (IGF) and 4.0 pg/µl of stem cell factor (SCF). Medium changes were carried out every other day by removing 5 µl of medium and adding 5 µl of 2X new medium. The latter assumed that growth factors will be labile after some period of continuous culture. However, the net result is that the concentration of growth factors is doubled. Hence, the final medium contains now the following growth factor concentrations: 0.0125 U/µl of leukemia inhibitory factor (LIF), 0.5 pg/µl of basic fibroblast growth factor (bFGF), 1.125 pg/µl of insulin like growth factor (IGF) and 8.0 pg/µl of stem cell factor (SCF). The range of growth factor concentrations described here promote the maintenance and proliferation of PGCs in continuous culture. However, PGCs survive and proliferate better at the highest end of the described growth factor concentrations.

Using these culturing conditions, PGCs form large, dense, loosely adherent clumps of cells (some of the clumps have several hundreds of cells in them) within 3 to 4 days after collection. At the end of 7 days the clumps start to have large numbers of dead cells and cellular debris surrounding them. PGC clumps survive up to four weeks before they become cell monolayers. At weeks 1, 2 and 3, clumps have been dissociated, stained with a vital dye DiI and transferred into recipient embryos. At all three time-points cells were found in the gonads of some of the recipient embryos. The number of cells and the number of embryos showing stained PGCs in the gonads were inversely proportional to the age of the PGCs culture.

### ANTIBODY TESTING PROCEDURE AND GROWTH OF CONTROL CELL LINES.

Gamma irradiated (8000 rads) STO feeder layer cells (American Type Culture Collection, Cat # 1503-CRL) were seeded on 4-well chamber slides at about 70 to 80% confluency in Dulbecco's Modified Eagle's Medium (DMEM; SIGMA, Cat # D-5523). Mouse ES cells, used as positive experimental controls, were seeded on to the STO feeder cell layers 8 to 10 hours later.

DMEM complete medium was prepared by supplementing the base medium to a final concentration of 4.5 g/l glucose (SIGMA, Cat # G-7021), 1.5 g/l Sodium bicarbonate (SIGMA, Cat # S-4019), 1 mM Sodium pyruvate (GIBCO, Cat # 11360-070), 0.1 mM 2β-mercaptoethanol (GIBCO, Cat # 21985-023), 10% fetal bovine serum (Hyclone, Cat # SH30070-03) and 1% antibiotic/antimycotic (SIGMA, Cat # A-7292).

Chicken fibroblasts seeded in 4-well chamber slides in DMEM complete medium were used as negative controls. Cells were incubated for 3 days at 37°C and 6% CO₂ when the mouse ES cells formed visible colonies. The medium was decanted, rinsed in phosphate buffered saline (PBS) and the cells were fixed in cold 4% paraformaldehyde (SIGMA, Cat # P-6148) for 15 minutes at 4°C.

Cell clusters from 98-day old chicken PGC cultures in vitro were transferred on to 4-well chamber slides and fixed in cold 4% paraformaldehyde, while fresh chicken PGCs were fixed on regular glass slides. Blocking was done for 30 minutes using blocking reagent (1 mg/ml bovine serum albumin in PBS, Fisher, Cat # BP1605-100) .

Antibodies were diluted at a rate of 5 µg/ml in the blocking reagent, and 200 µl was applied on the respective slides and incubated for 18 hours overnight at 4°C. Cells were rinsed once in cold PBS. Two hundred microliters of the secondary antibody (Fluorescein conjugated affinipure goat anti-mouse IgM, Jackson laboratories, Cat 9115-015-020), at a rate of 5 µg/ml in blocking reagent, were applied on each slide and incubated for a further one hour period at 37°C. Slides were washed three times for 5 minutes each in 4X Sodium Saline Citrate (SSC) containing 0.1 % Tween-20 (Fisher, Cat # BP337-100) at 37°C. Cells were stained for 10 minutes at room temperature in 2X SSC containing 400 ng/ml DAPI (SIGMA, Cat # D-9542), rinsed for 3 minutes in 2X SSC containing 0.05% Tween-20 and mounted in DABCO (SIGMA, Cat D-2522) antifade.

Slides were observed under a Nikon Eclipse E800 photomicroscope equipped with brightfield, DIC, phase and fluorescence optics including a 100-Watt mercury lamp epifluorecsence illumination with standard excitation/barrier filters. Cells were observed under the appropriate filter sets to detect FITC signals and DAPI stained nuclei. Phase contrast images of the cells were also obtained. Digitized images were captured using a CoolCam liquid cooled CCD camera (Cool Camera Company, Decatur, GA) using the Image-Pro® Plus version 3.0 software (Media Cybernetics, Silver Springs, NM) and stored on an Iomega® ZIP® drive. Images were processed using the Photoshop® 4.0 (Adobe) software and printed on glossy photography quality paper using the Epson Stylus-800 printer.

### PGC TRANSFER INTO RECIPIENT EMBRYOS.

For PGC transfer, the recipient egg was positioned horizontally under a dissecting scope. A small hole was pierced into the air space of the egg to lower the internal pressure of the egg and prevent leakage. A 10 mm window was opened on the ventral surface of the egg and ~ 1 ml of PBS with 4% antibiotic/antimitotic was injected through the hole to bring the embryo up until it was slightly less than flush with the egg shell window. To inject the PGCs, a 30 µm pipet was beveled and then pulled using a microforge to form a fine point with polished edges. Two hundred PGCs per embryo transfer, dissociated as described below, were picked up manually using a needle-pipette and a suction tube. Prior to transfer, and while in the pipette, PGCs were mixed with a 0.04% solution of trypan blue stain. The total injection volume per embryo was 2 µl. For the final step, the recipient embryo was positioned to reveal a portion of the marginal vein. The needle-pipette with the PGCs was inserted and the contents carefully expelled. The needle-pipette was held in place for a few seconds and then removed. Recipient eggs were sealed with 2 layers of surgical tape followed by paraffin wax coating of the entire area. Recipient eggs were then placed back into a rotating incubator and incubated until hatching.

### EVALUATION OF THE PGC PHENOTYPE.

Chicken PGCs are positive for periodic acid Schiff staining (PAS) and are claimed to be positive for alkaline phosphatase. However, there is no convincing evidence that chicken PGCs are positive for the latter. In the absence of an alternative enzymatic or molecular marker method to characterize chicken PGCs, their phenotype was evaluated by transferring cells to recipient embryos and evaluating their presence in the gonads of the developing embryo. This method required culturing the PGCs in 100 µg/ml DiI in a α-MEM medium and rinsing prior to transfer to recipient embryos. Twenty-four hours post-transfer recipient embryos were removed and placed under an inverted microscope. DiI labeled cells observed in the gonads were interpreted as successful PGC migration to the gonads and confirmation of retention of PGC characteristics. A second method to evaluate the retention of the PGC phenotype was pursued by letting recipient embryos go to hatching and then evaluate the presence of donor PGCs in their gonads after breeding.

### BREEDING STRATEGY FOR PGC EVALUATION.

Two breeding strategies were followed. The first strategy used recipient black feathered birds with possible genotype i/i, E/E, s/s, b/b and donor white feathered broiler type birds with genotype I/I, E/E, S/S, B/B. To prove that recipient animals were chimeric, that is to say that contain their own PGCs and donor PGCs in their gonads, they were mated to pure black feathered birds. If the resulting progeny was all black feathered then the animal was assumed to be non chimeric. However, if some of the progeny was white feathered with some black feathered patches then the recipient animal would be chimeric. For the second breeding strategy Bar Rock birds were used as recipient embryos while white feathered broiler type birds were continued to be used as donors. In this latter case when putative chimeric birds were mated to pure Bar Rocks, the presence of white feathered progeny with some barred feathers would identify a positive chimeric bird. Fifty progeny were obtained from each putative chimeric bird before concluding on its chimeric status.

### PROGENY TESTS.

Putative chimeric E/-(WB) birds when crossed to WB birds produced pure white chicks when they originated from a donor (WB) PGC and, white with black speckled feathers chicks when they originated from the (E/-) PGC. Similarly, when BR(WB) were crossed to WB birds, pure white chicks were produced when originating from a donor (WB) PGC and white-speckled black chicks when they originated from (BR) PGCs. Crosses between putative BR chimeric birds were also done. For the latter, white chicks were produced when fertilization between two (WB) PGCs occurred and black chicks were the result of fertilization with two (BR) PGC. The intermediate white chick with speckled black feathers only happened when a (BR) PGC was fertilized by a (WB) PGC.

### LONG-TERM CULTURES BEYOND 25 DAYS (EG CELLS).

After 25 days of continuous cultures, PGC clumps form rapidly spreading monolayers. These monolayers of cells have a flat adherent base and looser clumps and chains of PGC like cells on the upper surface. Some packets of these monolayers of cells remain PAS positive. DiI stained cells obtained from these monolayers have been transferred to recipient embryos. Some embryos have shown few cells localized in their gonads. Cell monolayers have been passaged successfully. Generally, these cells are capable of undergoing 3 to 5 passages before they start to slow down their proliferation, age and become fibroblastic looking. There are several cell lines that have gone through multiple passages and continue to thrive without apparent differentiation for about four months in continuous culture.

Two cell lines obtained from monolayers, P102896 and P110596, have been frozen. The former did not show apparent differentiation and was marginally positive for alkaline phosphatase while the latter showed neuronal cell morphology and was strongly positive for alkaline phosphatase. As discussed above, further characterizations of PGC monolayers as described herein (specifically the putative EG cells) will further confirm their totipotency and pluripotency.

### Summary of Results

Chimeric chickens were generated from fresh and cryopreserved PGCs. Twenty-five (74%) out of 34 putative chimeric chickens, produced with fresh PGCs transfers, proved to be true chimeric animals after progeny testing. Thirty (88%) out of 34 putative chimeric birds, produced with cryopreserved PGCs, were demonstrated to be true chimeric chickens. In all cases, at least 40 progeny were produced and the number of donor PGCs that were fertilized per chimeric bird varied from 1.4% to 100%, with the majority ranging between 30% to 60%. Assuming that the latter is a reflection of the number of PGCs that migrated to the gonad after injection, then the range of success per injection was varied. However, other mechanisms might be operating that might impact the number of PGCs that become established in the recipient gonad. Such mechanisms were not evaluated in this study. Also, on average, we did not observe any significant alteration of sex ratio in the progeny of chimeric birds.

### PGC culture conditions

None of the cell feeder layers evaluated in this study improved the long term culture conditions of the PGCs. None of the growth factors alone, at any of the concentrations studied, was able to sustain PGCs in vitro without differentiation. Combinations of two and three growth factors were also tested with little success. Based on our results, it appears that all of the factors described above (LIF, BFGF, IGF and SCF) are required for long term culture of PGCs. Based on DiI staining of PGCs we have observed that, under our culture conditions, PGCs originating from 14 day old continuous cultures migrate to the gonads of recipient embryos after injection. We have also transferred PGCs that have been maintained in culture for 25 days to three recipient embryos that were carried to hatch. One of these embryos was determined to be chimeric based on progeny testing results.

### PGC phenotype under long term culture conditions

After collection, PGCs are recognized by their size and by the presence of lipid droplets in their membrane and cytoplasm. At about 48 hours after collection, PGCs clump together and start dividing as evidenced by the growth in size of the clump and the number of cells observed after trypsin dissociation of the clump. Only PGCs that form clumps survive, all others die. Generally, a culture starting with 100 PGCs would end up with an average of 600 to 800 PGCs within seven days. Clearly some PGCs divide, albeit not at an efficient rate. However, as indicated above, these PGCs maintain their ability to migrate to the gonads.

### Long-Term Cultures Beyond 25 Days

After 25 days of continuous cultures, PGC clumps form rapidly spreading monolayers. These monolayers of cells have a flat adherent base and looser clumps and chains of PGC like cells on the upper surface. Some packets of these monolayers of cells remain PAS positive. DiI stained cells obtained from these monolayers have been transferred to recipient embryos. Some embryos have shown few cells localized in their gonads. Cell monolayers have been passaged successfully. Generally, these cells are capable of undergoing 3 to 5 passages before they start to slow down their proliferation, aged and become fibroblastic looking. There are a few cell lines that have gone through multiple passages and continue to thrive without apparent differentiation for about four months in continuous culture.

Two cell lines in particular obtained from monolayers have been frozen and are designated P102896 and P110596, although many cell lines having similar characteristics have been established. The former did not show apparent differentiation and was marginally positive for alkaline phosphatase while the latter showed neuronal cell morphology and was strongly positive for alkaline phosphatase.

In particular, it has been shown that PGCs cultured using the above four growth factors for at least 25 days can successfully colonize the gonads and produce chimeric chickens. Also, we have maintained PGC cells in culture for up to four months. These cultures still appear to comprise cells having the desired PGC phenotype based on the results of tests described herein. While these cells were not tested for their ability to produce chimeric birds, based on their appearance, it is expected that they should be useful therefor.

### Detection of EMA-1 and MC-480 antibodies on chicken PGCs in loag-term culture

Monoclonal antibodies EMA-1 and MC-480 were tested on mouse ES cells (positive controls), chicken PGCs that were in culture for 98 days, freshly collected chicken PGCS, and chicken fibroblast cells (negative controls).

EMA-1 antibody bound with high affinity to mouse ES cells (Figure 1), cells in 98-day old PGC cultures (Figure 2) and to most of the fresh chicken PGCs (Figures 3). EMA-1 did not bind to chicken fibroblasts (Figure 4). These results are in agreement with that of Hahnel and Eddy (1987) who reported that this antibody detected cell surface markers that are present on most of the pluripotent mouse embryonic cells as well as PGCs. They also reported that EMA-1 showed recurrent positive cells along the urogenital tract epithelia of adult tissues as well as early embryos. They did not report the detection of this epitope on any other adult tissue. It is possible that the antibody detects the epitope on adult urogenital ridge by virtue of the presence of germ cells. Pain et al (1996) reported the use of EMA-1 to identify chicken ES cells in culture. He suggested that EMA-1 epitope can be a useful marker to identify non-differentiated embryonic stem cells. In our experiments, EMA-1 gave very strong positive signals on 98-day old chicken PGC cultures comparable to the mouse ES cells, as well as on fresh PGCs. However, this antibody does not differentiate between PGC and ES cell phenotypes; it simply indicates the potential for pluripotency and totipotency. This suggests that PGCs in long term cultures either remain as PGCs or are de-differentiating to pluripotent EG cells.

MC-480 antibody reacted strongly with cell surface antigens on mouse EG cells (Figure 5) and 98-day old PGCs in culture (Figure 6). Very few fresh PGCs were positive for the antigen (Figures 7) while chicken fibroblasts were always negative (Figure 8). These results suggest that PGCs in long-term in vitro culture de-differentiate into pluripotent EG cells. The fact that some of the fresh PGCs gave positive signals with the antibody indicates that some fresh PGCs still retain some of the ES antigens during their migratory period to the embryonic gonads. These antigens may be lost subsequently. However, it is also possible that PGCs that exhibit the EG antigen on their surfaces eventually go on to survive better in our long-term cultures. The two results taken together suggest that PGCs in our long-term cultures de-differentiate to become pluripotent stem cells. This finding is similar to the report that mouse PGCs de-differentiate in culture and become EG cells (Matsui *et al*, 1992).

These results indicate that our chicken PGC culture medium influences the de-differentiation of chicken PGCs into EG cells. This is an important step in the production of pluripotent chicken cells useful for the efficient generation of transgenic animals and avian cloning.

### PGC transfection

Lipofection of a vector containing the green fluorescence protein reporter gene has been used for transfection of PGCs. On average 1/50 PGCs were transiently transfected, however, no stable transfected cell line has been developed yet.

In summary, these results indicate that PGCs can be maintained for long periods and successfully used for the production of chimeric birds. Further changes in growth factor concentrations and the use of other growth factors may further optimize culturing conditions. To be useful, a PGC culture system should allow for transfection and selection of PGCs while maintaining the PGC ability to migrate to the gonads. Also, these results indicate that avian (e.g., chicken) PGCs revert to the EG cell phenotype, as occurs with mouse PGCs (Matsui et al., Cell, 70:841-847, 1992). Therefore, injection of dispersed EG cells into recipient blastoderms should enable the generation of chimeric and transgenic chickens. Also, these cells are potentially useful for producing transgenic EG cell lines which can be used to produce transgenic chimeric and cloned avians.

## Claims

1. A culturing method which provides for the production of avian embryonic germ (EG) cells comprising the following steps:
(i) isolating primordial germ cells (PGCs) from a desired avian;
(ii) culturing said primordial germ cells in a culture medium containing at least the following growth factors contained in amounts sufficient to maintain said PGCs for prolonged periods in tissue culture:
(1) leukemia inhibitory factor (LIF),
(2) basic fibroblast growth factor (bFGF),
(3) stem cell factor (SCF), and
(4) insulin-like growth factor (IGF),
for prolonged time period sufficient to produce a culture having a compact multilayer like appearance; and
(iii)identifying embryonic germ cells contained therein.

2. The method of Claim 1, wherein the minimal amounts of said growth factors are :
(1) LIF (0.00625 U/µl),
(2) bFGF (0.25 pg/µl),
(3) IGF (0.5625 pg/µl), and
(4) SCF (4.0 pg/µl).

3. The method of Claim 2, wherein the maximal amounts of said growth factors range from about two times to one hundred times said minimum amounts.

4. The method of Claim 1, wherein said avian PGCs are obtained from an avian of the genus *Gallinacea*.

5. The method of Claim 4, wherein said PGCs are chicken PGCs or turkey PGCs.

6. The method of Claim 1, wherein said PGCs are maintained in culture for at least 25 days.

7. The method according to Claim 6, wherein said PGCs are maintained in culture for longer than 25 days.

8. The method according to Claim 7, wherein said PGCs are maintained in culture for at least 4 months.

9. The method of Claim 1, wherein avian EG cells are identified based on their expression of mouse-stage specific antigen 1, and/or reactivity with EMA-1 or MC-480 monoclonal antibody.

10. The method of Claim 9, wherein the EG phenotype of said cells is further confirmed by transferral of such cells to a suitable avian embryo.

11. The method of Claim 10, wherein said embryo is a stage X chicken embryo.

12. The method of Claim 1, which further comprises:
(iv) transfecting or transforming the resultant EG cells with a desired nucleic acid sequence

13. The method of Claim 12, wherein said nucleic acid sequence encodes a therapeutic polypeptide.

14. A method of producing chimeric avians comprising:
(i) isolating primordial germ cells (PGCs) from an avian;
(ii) maintaining such PGCs in a tissue culture medium containing at least the following growth factors:
(1) leukemia inhibitory factor (LIF),
(2) basic fibroblast growth factor (bFGF),
(3) stem cell factor (SCF), and
(4) insulin-like growth factor (IGF)for a sufficient time to produce embryonic germ (EG) cells;
(iii)transferring said EG cells into a recipient avian embryo; and
(iv) selecting for chimeric avians which have the desired PGC phenotype.

15. The method according to Claim 14, wherein said PGCs are derived from avian embryos of the genus *Gallinacea.*

16. The method according to Claim 15, wherein said avian embryos are turkey or chicken embryos.

17. The method according to Claim 14, wherein said EG cells are transfected or transformed with a desired nucleic acid sequence prior to transferral to a recipient avian embryo.

18. The method according to Claim 17, wherein said nucleic acid sequence encodes a therapeutic polypeptide.

19. The method according to Claim 18, which further includes purifying said therapeutic polypeptide from the eggs of the chimeric avians produced according to step (iv), or the systemic circulating system or body fluids or tissues.

20. The method according to Claim 14, wherein the PGCs are injected into the dorsal aorta of a recipient avian embryo or into recipient blastoderms.

21. An avian EG cell line obtained by the culturing method of Claim 1.

22. The cell line of Claim 21, which is a chicken or turkey EG cell line.

23. The cell line of Claim 21, which contains an inserted nucleic acid sequence.

## Patentansprüche

1. Kultivierungsverfahren, das die Erzeugung von Vogelembryonalkeimzellen (EG-Zellen) ermöglicht und die folgenden Schritte aufweist:
(i) Isolieren von primordialen Keimzellen (PGCs) von einem gewünschten Vogel;
(ii) Kultivierung der primordialen Keimzellen in einem Kulturmedium, das mindestens die folgenden Wachstumsfaktoren enthält, die in Mengen enthalten sind, die ausreichen, um die PGCs über längere Zeiträume in Gewebekultur zu halten:
(1) Leukämie-Hemmungsfaktor (LIF),
(2) basischer Fibroblast-Wachstumsfaktor (bFGF),
(3) Stammzellenfaktor (SCF) und
(4) insulinartiger Wachstumsfaktor (IGF),
über einen längeren Zeitraum, der ausreicht, um eine Kultur zu erzeugen, die ein kompaktes vielschichtartiges Aussehen hat; und
(iii) Identifizieren von darin enthaltenen Embryonalkeimzellen.

2. Verfahren nach Anspruch 1, wobei die Mindestmengen der genannten Wachstumsfaktoren sind:
(1) LIF (0,00625 U/µl),
(2) bFGF (0,25 pg/µl),
(3) IGF (0,5625 pg/µl) und
(4) SCF (4,0 pg/µl).

3. Verfahren nach Anspruch 2, wobei die maximalen Mengen der Wachstumsfaktoren im Bereich zwischen ungefähr dem Zweifachen bis zu dem Hundertfachen der Mindestmengen sind.

4. Verfahren nach Anspruch 1, wobei die Vogel-PGCs von einem Vogel der Gattung *Gallinacea* gewonnen werden.

5. Verfahren nach Anspruch 4, wobei die PGCs Huhn-PGCs oder Truthahn-PGCs sind.

6. Verfahren nach Anspruch 1, wobei die PGCs für mindestens 25 Tage in Kultur gehalten werden.

7. Verfahren nach Anspruch 6, wobei die PGCs länger als 25 Tage in Kultur gehalten werden.

8. Verfahren nach Anspruch 7, wobei die PGCs für mindestens 4 Monate in Kultur gehalten werden.

9. Verfahren nach Anspruch 1, wobei Vogel-EG-Zellen auf der Basis ihrer Expression von Mouse-Stage-spezifischem Antigen 1 und/oder ihrem Reaktionsvermögen mit EMA-1 oder MC-480 monoklonalem Antikörper identifiziert werden.

10. Verfahren nach Anspruch 9, wobei der EG-Phänotyp der Zellen ferner durch Transfer solcher Zellen auf einen geeigneten Vogelembryo bestätigt wird.

11. Verfahren nach Anspruch 10, wobei der Embryo ein Huhnembryo der Stufe X ist.

12. Verfahren nach Anspruch 1, das ferner aufweist:
(iv) Durchführen einer Transfektion oder Transformation der resultierenden EG-Zellen mit einer gewünschten Nukleinsäuresequenz.

13. Verfahren nach Anspruch 12, wobei die Nukleinsäuresequenz für ein therapeutisches Polypeptid codiert.

14. Verfahren zum Erzeugen von Chimärenvögeln, das aufweist:
(i) Isolieren von primordialen Keimzellen (PGCs) von einem Vogel;
(ii) Halten von solchen PGCs in einem Gewebekulturmedium, das mindestens die folgenden Wachstumsfaktoren enthält:
(1) Leukämie-Hemmungsfaktor (LIF),
(2) basischer Fibroblast-Wachstumsfaktor (bFGF),
(3) Stammzellenfaktor (SCF) und
(4) insulinartiger Wachstumsfaktor (IGF)
für eine ausreichende Zeit, um Embryonalkeimzellen (EG-Zellen) zu erzeugen;
(iii) Transferieren der EG-Zellen in einen Empfänger-Vogelembryo;
(iv) Selektieren für Chimärenvögel, die den gewünschten PGC-Phänotyp haben.

15. Verfahren nach Anspruch 14, wobei die PGCs von Vogelembryos der Gattung *Gallinacea* abgeleitet werden.

16. Verfahren nach Anspruch 15, wobei die Vogelembryos Truthahn- oder Huhnembryos sind.

17. Verfahren nach Anspruch 14, wobei die EG-Zellen vor dem Transfer auf einen Empfänger-Vogelembryo einer Transfektion oder Transformation mit einer gewünschten Nukleinsäuresequenz unterzogen werden.

18. Verfahren nach Anspruch 17, wobei die Nukleinsäuresequenz ein therapeutisches Polypeptid codiert.

19. Verfahren nach Anspruch 18, das ferner aufweist: Reinigen des therapeutischen Polypeptids aus den Eiern der gemäß Schritt (iv) erzeugten Chimärenvögel oder dem systemischen Kreislaufsystem oder Körperflüssigkeiten oder -gewebe.

20. Verfahren nach Anspruch 14, wobei die PGCs in die dorsale Aorta eines Empfänger-Vogelembryos oder in Empfänger-Blastoderme injiziert werden.

21. Vogel-EG-Zelllinie, die durch das Kultivierungsverfahren nach Anspruch 1 gewonnen ist.

22. Zelllinie nach Anspruch 21, die eine Huhn- oder Truthahn-EG-Zelllinie ist.

23. Zelllinie nach Anspruch 21, die eine eingefügte Nukleinsäuresequenz enthält.

## Revendications

1. Procédé de culture qui permet la production de cellules germinales embryonnaires d'oiseau (EG) comprenant les étapes suivantes :
(i) l'isolement de cellules germinales primordiales (PGC) d'un oiseau souhaité ; et
(ii) la culture desdites cellules germinales primordiales dans un milieu de culture contenant au moins les facteurs de croissance suivants, contenus en des quantités suffisantes pour conserver lesdites PGC pendant des périodes prolongées en culture tissulaire :
(1) le facteur inhibiteur de la leucémie (LIF),
(2) le facteur de croissance de fibroblastes basique (bFGF),
(3) le facteur des cellules souches (SCF), et
(4) le facteur de croissance de type insuline (IGF),
pendant une période prolongée suffisante pour produire une culture ayant une apparence de type couches multiples compactes ; et
(iii) l'identification des cellules germinales embryonnaires y étant contenues.

2. Procédé selon la revendication 1, dans lequel les quantités minimales desdits facteurs de croissance sont :
(1) LIF (0,00625 U/µL)
(2) bFGF (0,25 pg/µL)
(3) IGF (0,5625 pg/µL) et
(4) SCF (4,0 pg/µL)

3. Procédé selon la revendication 2, dans lequel les quantités maximales desdits facteurs de croissance se trouvent dans la plage d'environ 2 fois à 100 fois lesdites quantités minimales.

4. Procédé selon la revendication 1, dans lequel lesdites PGC d'oiseau proviennent d'un oiseau du genre *Gallinacea*.

5. Procédé selon la revendication 4, dans lequel lesdites PGC sont des PGC de poulet ou des PGC de dinde.

6. Procédé selon la revendication 1, dans lequel lesdites PGC sont conservées en culture pendant au moins 25 jours.

7. Procédé selon la revendication 6, dans lequel lesdites PGC sont conservées en culture pendant plus de 25 jours.

8. Procédé selon la revendication 7, dans lequel lesdites PGC sont conservées en culture pendant au moins 4 mois.

9. Procédé selon la revendication 1, dans lequel les cellules EG d'oiseau sont identifiées en se basant sur leur expression de l'antigène murin SSEA-1 (stage specific embryonic antigen), et/ou sur leur réactivité avec l'anticorps monoclonal EMA-1 ou MC-480.

10. Procédé selon la revendication 9, dans lequel Le phénotype EG desdites cellules est en outre confirmé par le transfert de ces cellules dans un embryon d'oiseau adapté.

11. Procédé selon la revendication 10, dans lequel ledit embryon est un embryon de poulet au stade X.

12. Procédé selon la revendication 1, qui comprend en outre :
(iv) la transfection ou la transformation des cellules EG résultantes avec une séquence d'acide nucléique souhaitée.

13. Procédé selon la revendication 12, dans lequel ladite séquence d'acide nucléique code pour un polypeptide thérapeutique.

14. Procédé de production d'oiseaux chimériques comprenant :
(i) l'isolement de cellules germinales primordiales (PGC) d'un oiseau ;
(ii) la conservation de ces PGC dans un milieu de culture tissulaire contenant au moins les facteurs de croissance suivants :
(1) le facteur inhibiteur de la leucémie (LIF),
(2) le facteur de croissance de fibroblastes basique (bFGF),
(3) le facteur des cellules souches (SCF), et
(4) le facteur de croissance de type insuline (IGF), pendant une durée suffisante pour produire des cellules germinales embryonnaires (EG) ;
(iii) le transfert desdites cellules EG à un embryon d'oiseau receveur ; et
(iv) le choix d'oiseaux chimériques qui ont le phénotype des PGC souhaité.

15. Procédé selon la revendication 14, dans lequel lesdites PGC proviennent d'embryons d'oiseaux du genre *Gallinacea*.

16. Procédé selon la revendication 15, dans lequel lesdits embryons d'oiseaux sont des embryons de dinde ou des embryons de poulet.

17. Procédé selon la revendication 14, dans lequel lesdites cellules EG sont transfectées ou transformées avec une séquence d'acide nucléique souhaitée avant le transfert à un embryon d'oiseau receveur.

18. Procédé selon la revendication 17, dans lequel ladite séquence d'acide nucléique code pour un polypeptide thérapeutique.

19. Procédé selon la revendication 18, qui comprend en outre la purification dudit polypeptide thérapeutique des oeufs des oiseaux chimériques produits selon l'étape (iv), ou du système circulatoire systémique ou des fluides ou tissus corporels.

20. Procédé selon la revendication 14, dans lequel les PGC sont injectées dans l'aorte dorsale d'un embryon d'oiseau receveur ou dans des blastodermes receveurs.

21. Lignée de cellules EG d'oiseau obtenue par le procédé de culture selon la revendication 1.

22. Lignée cellulaire selon la revendication 21, qui est une lignée de cellules EG de poulet ou de dinde.

23. Lignée cellulaire selon la revendication 21, qui contient une séquence d'acide nucléique insérée.
